(19) Europäisches Patentamt
European Patent Office
Office européen des brevets

(11) **EP 2 253 697 B1**

(12) **EUROPÄISCHE PATENTSCHRIFT**

(45) Veröffentlichungstag und Bekanntmachung des Hinweises auf die Patenterteilung:
**24.10.2012 Patentblatt 2012/43**

(21) Anmeldenummer: **10008161.1**

(22) Anmeldetag: **15.09.2006**

(51) Int Cl.:
*C11D 17/00* (2006.01)     *A61K 8/86* (2006.01)
*A61Q 19/00* (2006.01)     *B01F 17/00* (2006.01)
*A23L 1/035* (2006.01)     *A61Q 19/10* (2006.01)
*A61K 9/107* (2006.01)     *C11D 1/83* (2006.01)
*C11D 1/835* (2006.01)     *C11D 1/825* (2006.01)
*A61K 8/06* (2006.01)

(54) **Verfahren zur Aufweitung des Temperaturfensters in Mikroemulsionen mittels Additiven**

Method for expanding the temperature range in microemulsions using additives

Procédé d'élargissement de la fenêtre de température dans des microémulsions à l'aide d'additifs

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **18.10.2005 DE 102005049765**

(43) Veröffentlichungstag der Anmeldung:
**24.11.2010 Patentblatt 2010/47**

(62) Dokumentnummer(n) der früheren Anmeldung(en) nach Art. 76 EPÜ:
**06791384.8 / 1 937 795**

(73) Patentinhaber: **Forschungszentrum Jülich Gmbh (FJZ)**
**52425 Jülich (DE)**

(72) Erfinder:
• **Allgaier, Jürgen**
**52074 Aachen (DE)**
• **Frank, Christian**
**50169 Horrem (DE)**
• **Frielinghaus, Henrich**
**85375 Neufahrn (DE)**
• **Richter, Dieter**
**52428 Jülich (DE)**

(56) Entgegenhaltungen:
WO-A1-2004/103542     WO-A2-00/12660
DE-A1- 19 641 672     US-A- 5 602 090

Bemerkungen:
Die Akte enthält technische Angaben, die nach dem Eingang der Anmeldung eingereicht wurden und die nicht in dieser Patentschrift enthalten sind.

EP 2 253 697 B1

**Beschreibung**

Die Erfindung betrifft ein Verfahren zur Aufweitung des Temperaturfensters in Mikroemulsionen mittels Additiven.

Nach dem Stand der Technik sind Verfahren bekannt, mit denen die Effizienz von Tensiden, insbesondere in Mikroemulsionen aber auch in Emulsionen, erhöht werden können. Hierzu werden Additive zugegeben, welche AB-Blockcopolymere mit verschiedenen Blöcken A und B sind. Die deutsche Patentanmeldung 198 39 054.8-41 und die WO 00/12660A2 offenbaren ein Verfahren zur Effizienzsteigerung von Tensiden bei simultaner Unterdrückung lamellarer Mesophasen, ein Verfahren zur Stabilisierung der Temperaturlage des Einphasengebietes für Öl-, Wasser-Tensidmischungen, ein Verfahren zur Vergrößerung der Strukturgröße von emulgierten Flüssigkeitsteilchen in Mikroemulsionen sowie ein Verfahren zur Verminderung der Grenzflächenspannung von Öl- Wassergemischen, bei denen AB-Blockcopolymere mit einem wasserlöslichen Block A und einem wasserunlöslichen Block B zugegeben werden. Die Polymere bestehen aus einem wasserlöslichen Block A und einem wasserunlöslichen Block B. Die unteren Grenzen der Molekulargewichte für A und B liegen bei 500 g/mol. Dieses Verfahren eignet sich für Mikroemulsionen.

Die DE 10 2004 058 956.9 beschreibt ein Verfahren zur Steigerung der Effizienz von Tensiden und Emulgatoren in Emulsionen und Mikroemulsionen durch Zugabe von Additiven, dadurch gekennzeichnet, dass dem Tensid oder Emulgator als Additiv ein Polyalkylenoxid-Blockcopolymer mit einem wasserlöslichen Block A und einem öllöslichen Block B zugegeben wird. Die unteren Grenzen der Molekulargewichte für A und B liegen bei 1.000 g/mol.

Die deutsche Patentanmeldung 10 2005 023 762.2-43 beschreibt ein Verfahren zur Effizienzsteigerung von Tensiden in Mikroemulsionen, die Siliconöl enthalten, mit Hilfe von AB-Blockcopolymeren. Die AB-Blockcopolymere bestehen aus einem wasserlöslichen Block A und einem Block B, der entweder ein Polyalkylenoxid mit mindestens vier C-Atomen im Monomerbaustein oder ein Polydien oder ein teilweise oder vollständig hydriertes Polydien oder ein Polyalkan ist. Die unteren Grenzen der Molekulargewichte für A und B liegen bei 500 g/mol.

Die Offenlegungsschrift DE 103 23 180 A1 beschreibt Mischungen, enthaltend ein Tensid und ein Cotensid, dadurch gekennzeichnet, dass man als Cotensid ein amphiphiles Kammpolymer einsetzt, aufweisend ein Rückgrat mit am Rückgrat angebrachten zwei oder mehreren Seitenketten, wobei sich die Seitenketten untereinander und/oder die Seitenketten vom Rückgrat in ihrem amphiphilen Charakter unterscheiden. Das Cotensid eignet sich zur Effizienzsteigerung in Mikroemulsionen.

Es besteht Bedarf, Verfahren

- mit einfach herstellbaren Additiven,
- mit Bestandteilen, deren biologische Abbaubarkeit möglichst gut ist,
- die universell einsetzbar sind, so dass die hydrophobe Komponente im Additiv nicht dem verwendeten Öl angepasst werden muss,

zur Verfügung zu stellen.

Das Verfahren soll insbesondere für Kohlenwasserstoffe, Siliconöle, "polare" Öle wie Ester, die ihrerseits gute biologische Abbaubarkeit besitzen, geeignet sein. Neben Kostengründen ist die Tensideinsparung auch aus ökologischen oder gesundheitlichen Gründen vorteilhaft. Bei Siliconölmikroemulsionen sind diese Bedürfnisse besonders ausgeprägt, da die hier verwendeten Silicontenside sehr teuer sind oder die konventionellen Tenside in sehr hohen Konzentrationen eingesetzt werden müssen. Ein weiterer Vorteil der Einsparung von Tensiden kann gegeben sein, wenn sich Tenside bei der Anwendung der Mikroemulsion störend auswirken. Beispielhaft können Körperpflegeprodukte genannt werden, deren Tensidgehalt aufgrund der Haut schädigenden Wirkung von Tensiden möglichst gering sein sollte. Weitere Beispiele sind Mikroemulsionen, die verwandt werden, um Filme auszubleichen, die möglichst wasserresistent sein sollen.

Es ist daher die Aufgabe der Erfindung, ein Verfahren zur Aufweitung des Temperaturfensters des Mikroemulsionsbereichs zur Verfügung zu stellen.

Ausgehend vom Oberbegriff der Ansprüche 1 und 2 wird die Aufgabe erfindungsgemäß gelöst mit den im kennzeichnenden Teil der Ansprüche 1 und 2 angegebenen Merkmalen.

Mit dem erfindungsgemäßen Verfahren und der Zusammensetzung ist es nunmehr möglich, das Temperaturfenster aufzuweiten.

Vorteilhafte Weiterbildungen der Erfindung sind in den Unteransprüchen angegeben.

In den Figuren sind Beispiele für das Verhalten der erfindungsgemäß eingesetzten Polymere dargestellt.

Es zeigt:

Fig. 1:    Mögliche Strukturen der polymeren Additive

Fig. 2:    Phasenverhalten des Systems Wasser - n-Dekan - $C_{10}E_4$ - $C_{12}E_{90}$

Fig. 3:     Phasenverhalten des Systems Wasser - n-Dekan - $C_{10}E_4$ - $C_{12}E_{190}$

Fig. 4:     Phasenverhalten des Systems Wasser -

Fig. 5:     n-Dekan - $C_{10}E_4$-$C_{12}E_{480}$ Phasenverhalten des Systems Wasser - n-Dekan - $C_{10}E_4$-Tergitol 15S30

Fig. 6:     Phasenverhalten des Systems Wasser - Hydroseal G232H -IMBENTIN AG 100/040 - Brij 700

Fig. 7:     Phasenverhalten des Systems Wasser - n-Dekan - $C_{10}E_4$ - $C_{18}E_{80}$.

Fig. 8:     Phasenverhalten des Systems Wasser - n-Dekan - $C_{10}E_4$ - $C_{18}E_{180}$

Fig. 9:     Phasenverhalten des Systems Wasser - n-Dekan - $C_{10}E_4$ - Berol EP35

Fig. 10:    Phasenverhalten des Systems Wasser - n-Dekan - $C_{10}E_4$ - $C_8E_{90}$.

Fig. 11:    Phasenverhalten des Systems Wasser - n-Dekan - $C_{10}E_4$ - IGEPAL DM 970

Fig. 12:    Phasenverhalten des Systems Wasser - n-Dekan - $C_{10}E_4$ - $C_{12}(E_{90})_2$

Fig. 13:    Phasenverhalten des Systems Was- ser/NaCl - n-Dekan - AOT - Brij 700

Fig. 14:    Phasenverhalten des System Was- ser/NaCl - Rapsmethylester - AOT - Brij 700

Fig. 15:    Phasenverhalten des Systems Wasser - MDM - $C_4D_3E_8$ - Brij 700

Fig. 16:    Phasenverhalten des Systems Wasser - n-Dekan - $C_{10}E_4$ - Brij 700

Fig. 17:    Phasenverhalten des Systems Wasser - n-Dekan-$C_{10}E_4$-Brij 700

Erfindungsgemäß werden zur Lösung der gestellten Aufgabe polymere Additive eingesetzt, welche aus mindestens einer wasserlöslichen Einheit bestehen, die mindestens an einem Kettenende mindestens eine hydrophobe Einheit besitzt und/oder eine hydrophobe Einheit als nichtterminalen Substituenten besitzt und/oder mindestens eine hydrophobe Einheit besitzen, welche zwischen den wasserlöslichen Einheiten des Polymers eingebaut ist.

Im gesamten polymeren Additiv überwiegt der hydrophile Charakter. Auf Grund der hydrophoben Einheit oder Einheiten bilden die Polymere in Wasser bevorzugt Micellen.

Die wasserlösliche Einheit des polymeren Additivs ist in ihrer Ausgestaltung nicht auf bestimmte Strukturtypen begrenzt, vielmehr kommt es erfindungsgemäß auf die Kombination der größeren wasserlöslichen Einheit mit der oder den hydrophoben Einheiten an.

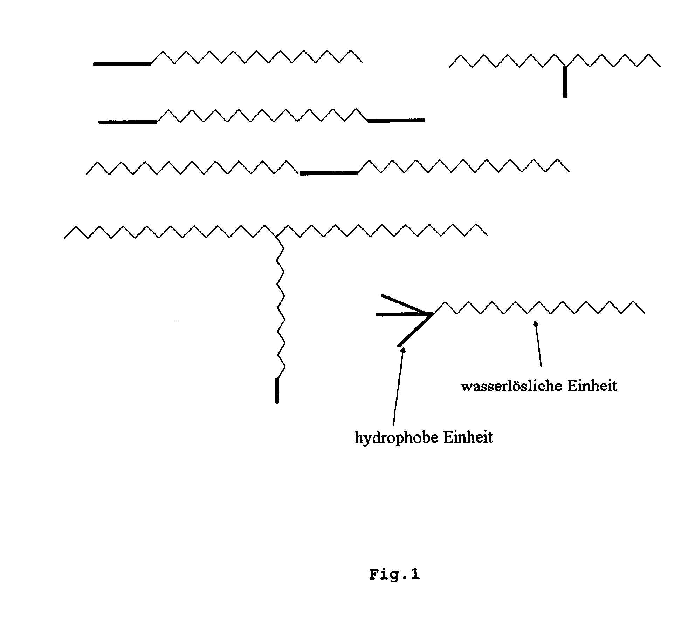

Die wasserlösliche Einheit des Polymers ist vorzugsweise linear, es sind aber auch sternförmige, verzweigte oder andere Strukturtypen möglich, wie in Figur 1 beispielhaft dargestellt ist. Unter linear wird bei Polymeren verstanden, dass die Atome, die das Rückgrat der Kette bilden, eine lineare Einheit darstellen. In Figur 1 sind die hydrophoben Einheiten durch fette Linien und die wasserlöslichen Einheiten durch Zickzacklinien dargestellt.

Die wasserlösliche Einheit kann nichtionisch oder ionisch, das heißt ein Polyelektrolyt sein. Die elektrischen Ladungen können sich an jedem Teil der wasserlöslichen Komponente des Polymers befinden. Es sind auch Strukturen denkbar, welche sich aus mindestens einem ionischen und einem nichtionischen Anteil zusammensetzen.

Beispielhaft, aber nicht beschränkend, können die wasserlöslichen Einheiten aus folgenden Monomeren oder deren Mischungen von mindestens zwei Komponenten bestehen: Ethylenoxid, Vinylpyrrolidin, Acrylsäure, Methacrylsäure, Maleinsäureanhydrid und Acrolein.

Der wasserlösliche Teil des polymeren Additivs ist bevorzugt ein Polyethylenoxid oder Polyethylenglykol. Weitere Beispiele sind Copolymerisate aus Ethylenoxid und Propylenoxid, Polyacrolein, Polyvinylalkohol und dessen wasserlösliche Derivate. Außerdem eignen sich Polyvinylpyrrolidon, Polyvinylpyridin, Polymaleinsäureanhydrid, Polymaleinsäure, Polyacrylsäure, Polymethacrylsäure, Polystyrolsulfonsäure und deren wasserlösliche Salze.

Die wasserlöslichen Einheiten sind vorzugsweise linear.

Die Molekulargewichtsverteilung der wasserlöslichen Einheit, definiert durch das Verhältnis des gewichtmittleren Molekulargewichts und des zahlenmittleren Molekulargewichts, beträgt bevorzugt ≤ 1,2.

Das zahlenmittlere Molekulargewicht der wasserlöslichen Einheit des polymeren Additivs liegt vorzugsweise zwischen 500 und 100000 g/mol, besser 2000 bis 20000 g/mol, besonders bevorzugt zwischen 3000 und 10000 g/mol.

In ähnlicher Weise wie für den wasserlöslichen Teil des polymeren Additivs ist die Ausgestaltung der hydrophoben Einheit nicht auf ausgewählte Strukturtypen beschränkt. Vielmehr kommt es auch hier lediglich auf die hydrophoben bzw. nicht-wasserlöslichen Eigenschaften dieser Einheit an.

Bevorzugte Molekülgrößen für die hydrophobe Einheit liegen bei 80 bis 1000 g/mol, besonders bevorzugt 110 bis 500 g/mol, insbesondere bevorzugt 110 bis 280 g/mol.

Die hydrophoben Einheiten bestehen aus nicht wasserlöslichen Resten. Dabei handelt es sich bevorzugt um Alkylreste, die bevorzugt zwischen 6 und 50 Kohlenstoffatome, besonders bevorzugt zwischen 8 und 20 Kohlenstoffatome enthalten. Die Reste können auch aromatische Gruppen oder Kohlenstoff- Doppel- oder Dreifachbindungen enthalten, sie können linear oder verzweigt sein. Außer Kohlenwasserstoffresten sind auch beliebige andere hydrophobe organische Reste verwendbar, die beispielsweise Sauerstoff, Stickstoff, Fluor oder Siliciumatome enthalten. Die hydrophobe Einheit kann auch ein Polymerisat sein.

Die hydrophobe Einheit kann ein Rest mit definierter Struktur und Molekulargewicht sein, wie beispielsweise Alkylreste. Auch Stoffgemische, wie sie beispielsweise in technischen Produkten vorkommen, sind möglich. Es kann sich aber auch um einen polymeren Rest handeln, wie Polybutylenoxid.

Die wasserlösliche Einheit des Polymers trägt an mindestens einem Kettenende eine hydrophobe Einheit.

An jedem Kettenende sind auch mehr als eine hydrophobe Einheit möglich.

Die wasserlösliche Einheit des Polymers kann eine hydrophobe Einheit in einer Nicht-Kettenend-Position tragen.

Weiterhin können hydrophobe Einheiten des polymeren Additivs an mindestens einer Stelle zwischen die wasserlöslichen Einheiten eingebaut sein, so dass die wasserlöslichen Einheiten des Polymers durch hydrophobe Einheiten unterbrochen werden.

Es sind alle Kombinationen der angeführten Strukturtypen möglich.

Das Verhältnis der Molekulargewichte von wasserlöslichem Teil zu hydrophobem Teil beträgt 2 - 1000, vorzugsweise 5 - 200, besonders bevorzugt 10 - 50.

In der bevorzugten Form ist die wasserlösliche Einheit des Additivs ein lineares Polymer und trägt an einem Kettenende eine hydrophobe Einheit.

Beispielhaft können folgende erfindungsgemäße polymere Additive aufgeführt werden:

- durch Ethoxylierung von $C_8$-$C_{20}$ - Alkoholen erhaltene Alkylethoxylate,

- durch Ethoxylierung von $C_{10}$-$C_{20}$ 1,2-Diolen erhaltene Alkylethoxylate,

- durch Ethoxylierung von $C_8$-$C_{20}$ $\alpha$, $\omega$- Diole erhaltene Alkylethoxylate,

- an beiden Kettenenden hydrophob modifiziertes Polyethylenglokol, das z. B. durch Umsetzung von Polyethylenglykol mit $C_8$-$C_{20}$ Isocyanaten oder $C_8$-$C_{20}$-Säurechloriden erhalten werden kann,

- AB Diblockcopolymere, ABA oder BAB Triblockcopolymere aus 1,2 Butylenoxid und Ethylenoxid.

Aufgrund der hydrophoben Einheiten bilden die Additive in Wasser bevorzugt Micellen.

In einer Ausführungsform befindet sich an jeweils beiden Enden der wasserlöslichen Einheit eine hydrophobe Einheit.

Als erfindungsgemäße Additive werden lineare wasserlösliche Polymere, die nur an einem Kettenende eine hydrophobe Einheit tragen, bevorzugt. Innerhalb dieses Strukturtyps werden Alkoholethoxylate bevorzugt, die einen hohen Ethoxylierungsgrad besitzen. Diese Substanzen können als Polyethylenoxid mit einem hydrophoben Alkylrest betrachtet werden oder als langkettige oder hydrophile Emulgatoren angesehen werden. Als hydrophobe Komponenten können beispielsweise aliphatische Alkohole oder Alkylphenole verwendet werden, die bevorzugt 8-20 Kohlenstoffatome besitzen. Die Alkoholethoxylate enthalten pro Mol Alkohol bevorzugt 25 bis 500 Mol, besonders bevorzugt 50-200 Mol Ethylenoxid. Ein Beispiel ist die kommerziell erhältliche Verbindung Brij 700 der Firma Uniqema.

Im polymeren Additiv sollte der Anteil der wasserlöslichen Einheiten, die nicht mit hydrophoben Einheiten verknüpft sind, möglichst gering sein, das heißt beispielsweise ≤ 20 Gew.%.

Beispielhaft aber nicht beschränkend können folgende Tenside und deren Gemische mit den erfindungsgemäßen Additiven verwendet werden:

Nichtionische Tenside der Klasse alkoxylierten Alkohole, z. B. Alkylethoxylate, auch solche mit einer engen Molekulargewichtsverteilung und/oder einem geringen Restalkoholgehalt, Alkylphenolethoxylate.

Sorbitanester und ethoxylierte Sorbitanester.

Nichtionische Tenside der Klasse Alkylpolyglucoside (APG, "Zuckertenside",) mit hydrophobem Cotensid.

Siliconpolyether-Tenside.

Anionische Tenside, z. B. Alkylsulfate, Alkylsulfonate, Alkylbenzolsulfonate, Alkylethersulfate, Sulfosuccinate, Alkylethercarboxylate, Phosphate und Carbonsäuresalze. Die anionischen Tenside werden bevorzugt in Form ihrer $Li^+$, $Na^+$, $K^+$, bzw. Ammonium - Salze verwendet.

Kationische Tenside, z.B.Tetraaklyammoniumverbindungen.

Amphotere Tenside, z. B. Sulfobetaine, Betaine, Amphoacetate, Amphopropionate.

Gemische aus Tensiden, insbesondere nichtionisch/anionisch oder nichtionisch/kationisch oder Silicontensid mit nicht-siliciumhaltigem Tensid.

Das erfindungsgemäß verwendete Polymere Additiv kann den gleichen Strukturtyp wie die in der Mikroemulsion verwendeten Tenside haben, jedoch muss das Molekulargewicht der hydrophilen wasserlöslichen Einheit des polymeren Additivs größer sein als das Molekulargewicht der hydrophilen Komponente des Tensids. Bevorzugt ist, wenn das Molekulargewicht der wasserlöslichen Einheit des polymeren Additivs mindestens 2 mal, besonders bevorzugt mindestens 5 mal oder mindestens 10 mal so groß ist, wie die hydrophile Komponente des Tensids.

Die wässrige Phase der Mikroemulsion kann Additive wie Salze oder wasserlösliche organische Verbindungen enthalten, wie z. B. Glycole.

Auch die Ölphase kann Additive enthalten, jedoch sollen die Additive nicht die Mikroemulsion zerstören.

Beispielsweise kann dem Wasser Glycerin zugegeben werden, um so den Brechungsindex der wässrigen Komponente dem der Ölkomponente anzugleichen. Dadurch werden effizienzgesteigerte Mikroemulsionen, die optisch trüb sind, wieder transparent. Dieses Verfahren ist insbesondere für Mikroemulsionen, die im Bereich Kosmetika, Haar- und Körperpflegeprodukte eingesetzt werden, von Bedeutung.

Die erfindungsgemäßen Mikroemulsionen müssen nicht grundsätzlich flüssig sein. Es werden darunter auch gelartig-feste Gemische verstanden, sofern es sich im thermodynamischen Sinne um eine Mikroemulsion handelt. Die feste Form kann daher erhalten werden, z. B. durch Zugabe von Additiven zur wässrigen und/oder ölartigen Komponente oder durch in der Mikroemulsion vorhandene Mesophasen.

Die Mikroemulsion kann bei tiefen Temperaturen fest werden, z. B. durch Festwerden der Ölphase.

Das Volumenverhältnis von wässriger Phase zu der ÖlPhase beträgt beispielsweise 0,01-100, bevorzugt 0,1-10 oder 0,3-3.

Der Massenanteil polymeres Additiv im Tensid-Additivgemisch beträgt beispielsweise bevorzugt 0,01-0,3, besonders bevorzugt 0,05-0,15.

Der Massenanteil Tensid/Additivgemisch in der Mikroemulsion beträgt bevorzugt 0,01-0,3, besonders bevorzugt 0,05-0,2. Bei größeren Temperaturfenstern ist oft ein höherer Massenanteil Tensid/Additiv-Gemisch in der Mikroemulsion nötig.

Von der Erfindung sind auch Mikroemulsionen mit den stofflichen Merkmalen der Offenbarung umfasst, welche dadurch charakterisiert sind, dass die Mikroemulsionen ein polymeres Additiv enthalten, wobei das Massenverhältnis von polymerem Additiv zu Tensid + Additiv $\leq 0,2$ beträgt und das Massenverhältnis von Tensid + polymerem Additiv zu Öl $\leq 0,5$ beträgt.

weiterhin ist es bevorzugt, wenn diese Mikroemulsionen dadurch gekennzeichnet sind, dass das Massenverhältnis von polymerem Additiv zu Tensid + polymerem Additiv $\leq 0,15$, oder $\leq 0,10$ beträgt.

Besonders bevorzugt beträgt dass Massenverhältnis von Tensid + polymerem Additiv zu Öl $\leq 0,33$.

Weitere erfindungsgemäße Mirkoemulsion, die die in der Offenbarung enthaltenen polymeren Additive enthalten, sind **dadurch gekennzeichnet, dass** das Massenverhältnis von polymerem Additiv zu Tensid und polymerem Additiv $\leq 0,2$ ist und dass das polymere Additiv an einer Stelle im Molekül eine oder mehrere hydrophobe Einheiten besitzt.

Vorteilhaft ist dabei, wenn das Massenverhältnis von polymerem Additiv zu Tensid und polymerem Additiv $\leq 0,15$, oder $\leq 0,10$ beträgt. Weiterhin bevorzugt ist, wenn das Massenverhältnis von Tensid + polymerem Additiv zu Öl $\leq 1$ ist oder wenn das Massenverhältnis von Tensid + polymerem Additiv zu Öl $\leq 0,5$, bevorzugt $\leq 0,33$ beträgt.

Die Aufweitung des Temperaturfensters hängt vom Massenanteil polymeren Additivs im Tensid-Additivgemisch sowie vom Molekulargewicht der wasserlöslichen Einheiten ab.

Die Aufweitung des Temperaturfensters wird in der Regel mit zunehmendem Massenanteil polymeren Additivs im Tensid-Additivgemisch sowie zunehmendem Molekulargewicht der wasserlöslichen Einheiten größer. Die Zunahme der Aufweitung des Temperaturfensters mit zunehmendem Molekulargewicht wird jedoch geringer ab Molekulargewichten, die je nach Fall zwischen 4000 und 20000 g/mol pro wasserlöslicher Einheit liegen können. Dies gilt besonders für höhere Tensidkonzentrationen.

Die oben genannten Werte sind in der Regel gültig. Jedoch können Abweichungen auftreten, die vom verwendeten polymeren Additiv, Tensid, Öl bzw. anderen Additiven abhängen.

Bei einem großen Verhältnis von Wasser zu Öl existieren bevorzugt Öl-in-Wasser tröpfchenförmige Mikroemulsionen.

Bei einem kleinen Verhältnis von Wasser zu Öl existieren bevorzugt Wasser-in-Öl tröpfchenförmige Mikroemulsionen.

Bei einem ausgeglichenen Verhältnis von Wasser und Öl existieren bevorzugt bikontinuierliche Mikroemulsionen.

Erfindungsgemäß wird durch die Zugabe der erfindungsgemäßen polymeren Additive zur Wasser-Öl-Tensid-Mischung, das Temperaturfenster erweitert.

Die Temperaturlage des Einphasengebiets wird durch die erfindungsgemäßen polymeren Additive verändert. Die Veränderung ist überraschenderweise abschätzbar. Bei Verwendung von nichtionischen Tensiden wird die Lage des Einphasengebiets zu höheren Temperaturen verschoben, bei Verwendung von ionischen Tensiden findet in der Regel eine Veränderung zu tieferen Temperaturen statt. Durch die Wahl geeigneter Tenside lässt sich der gewünschte Temperaturbereich einstellen.

Im Folgenden sollen einige Begriffe definiert werden:

Unter Mikroemulsionen werden Gemische umfassend Wasser, Öl und Tensid und ggf. Additive in der wässrigen und/oder Ölphase verstanden, welche thermodynamisch stabil sind.

Als Öl wird eine nicht mit Wasser mischbare Flüssigkeit verstanden. Dabei kann es sich um unter Druck verflüssigbare Gase oder um überkritische Fluide handeln, die bei Normaldruck gasförmig sind. Es werden auch Mikroemulsionen unter erhöhtem Druck von der Erfindung umfasst.

In Mikroemulsionen werden als Öle oft Kohlenwasserstofföle verwendet. Es sind aber auch Mikroemulsionen mit anderen Ölen wie Esterölen oder Siliconölen bekannt, die für das erfindungsgemäße Verfahren eingesetzt werden können.

Unter Aufweitung des Temperaturfensters wird verstanden, dass bei gleicher Gesamt-Tensidkonzentration der Temperaturbereich, in dem eine einphasige Mikroemulsion existiert, durch die Zugabe des erfindungsgemäßen polymeren Additivs größer ist, als ohne polymeres Additiv.

Das Temperaturfenster, innerhalb dem die Mikroemulsion stabil ist, wird aufgeweitet.

Zu den möglichen Anwendungen zählen Haar- und Körperpflegeprodukte und Kosmetika wie Deodorants, Hautpflegemittel, Sonnencremes, Lotionen, Shampoos, Duschgele, Badezusätze, Schmierstoffe, Gleitmittel, Trennmittel, Pflanzenschutzmittel, Arzneimittel, Lebensmittel, Lebensmitteladditive, Textilpflege und -hilfsmittel, Leder- und Pelzpflegemittel, Fahrzeugpflegemittel, Reiniger sowie Polituren, Einsatzstoffe für Haushalt, gewerbliche und industrielle Anwendungen, Hydraulikflüssigkeiten, Desinfektionsmittel, Lacke und Farben, Bauhilfsstoffe, Druckertinten, Sprengstoffe, Reinigungsmittel für Haushalt, Gewerbe und industrielle Anwendung. Damit wird eine Möglichkeit geschaffen, Mikroemulsionen herzustellen, deren Größe der emulgierten Flüssigkeitsteilchen denen von Emulsionen entsprechen. Das Temperaturfenster der Stabilität der Mikroemulsionen wird bei gleichem Tensidgehalt vergrößert werden, auch wenn Siliconöle zugegen sind.

Die erfindungsgemäßen Mikroemulsionen können auch als Reaktionsmedien verwendet werden, sie können hydrophobe Verunreinigungen aufnehmen oder sich durch die Aufnahme der hydrophoben Verunreinigungen bilden, beispielsweise bei der Verwendung als Wasch- oder Reinigungsmittel. Die erfindungsgemäßen Mikroemulsionen können auch hydrophobe Bestandteile abgeben, und/oder feste oder flüssige Oberflächen benetzen. Die erfindungsgemäßen Mikroemulsionen können auch in Form von Konzentraten vorliegen, die nach Verdünnung, beispielsweise mit Wasser, immer noch Mikroemulsionen sind. Bei den erfindungsgemäßen Mikroemulsionen kann es sich auch um zwei- oder dreiphasige Systeme handeln, wobei eine Mikroemulsionsphase mit überschüssiger Öl- und/oder Wasserphase koexistiert. Durch die Zugabe des erfindungsgemäßen Additivs wird jedoch gegenüber der Mischung ohne Additiv der Anteil der Mikroemulsionsphase erhöht.

Die Mikroemulsionen sind ohne großen Eintrag von Energie herstellbar. Die Komponenten können in beliebiger Reihenfolge gemischt werden, wobei es aufgrund der in der Regel guten Wasserlöslichkeit des polymeren Additivs vorteilhaft ist, das Polymer in Wasser vorzulösen oder direkt in das Wasser-Öl-Tensid-Gemisch zu geben.

Das erfindungsgemäß eingesetzte polymere Additiv kann auch als Gemisch mit einem Tensid bereitgestellt werden.

Die mittels der erfindungsgemäßen Zugabe der wasserlöslichen Polymere hergestellten Mikroemulsionen weisen emulgierte Flüssigkeitsvolumina auf, die denen von Emulsionen entsprechen können.

Mit der Effizienzsteigerung ist häufig eine Aufweitung des Temperaturintervalls verbunden, innerhalb dessen die Mikroemulsion thermodynamisch stabil ist. Dies ist besonders für technische Anwendungen von Vorteil, wo eine Stabilität über große Temperaturbereiche gewährleistet sein muss.

**Vorteile der Additive**

- Die polymeren Additive sind schon kommerziell erhältlich und sind kostengünstig herstellbar. In ihrer einfachsten Ausführungsform handelt es sich um hochethoxylierte Alkohole. Ein Beispiel ist Brij 700 (Uniqema).
- Sie sind in der Regel besser biologisch abbaubar als Polymere, die langkettige hydrophobe Segmente enthalten. Dies ist insbesondere hinsichtlich der immer strenger werdenden gesetzlichen Anforderungen an die Abbaubarkeit wichtig.
- Die polymeren Additive sind gut wasserlöslich und können problemlos in wässrigen Vorprodukten der Mikroemulsion gelöst werden.
- Die polymeren Additive sind universell einsetzbar, d. h. unabhängig vom chemischen Aufbau des Öls. Das gleiche Additiv kann somit in Mikroemulsionen eingesetzt werden, die sehr unterschiedliche Öle enthalten. Bei Additiven, die als hydrophobe Komponenten höhermolekulare polymere Einheiten enthalten, ist hingegen mehr auf die Verträglichkeit der hydrophoben Additivkomponente mit dem Öl zu achten. Beispielsweise eignen sich für Mikroemulsionen, die als Öl Siliconöl enthalten, nur Blockcopolymere, die bestimmte hydrophobe Blöcke enthalten.

Die genannten Vorteile gelten weitgehend auch für polymere Additive, die als Blockcopolymerisate vorliegen, da die hydrophoben Einheiten kurzkettig sind.

Die erfindungsgemäß eingesetzten wasserlöslichen Polymere eignen sich für bikontinuierliche, Wasser-in-Öl sowie Öl-in-Wasser Mikroemulsionen. Sie eignen sich für Mikroemulsionen, die als Ölkomponente Kohlenwasserstoffe enthalten, sie eignen sich aber auch für Mikroemulsionen, die als Ölkomponente polarere Öle wie Esteröle, Siliconöle oder überkritische Flüssigkeiten enthalten.

Die polymeren Additive eignen sich besonders für Mikroemulsionen mit ionischen Tensiden, die Einphasengebiete bei sehr hohen Temperaturen ausbilden. Durch Zugabe des polymeren Additivs wird der Temperaturbereich des Einphasengebiets zu tieferen Temperaturen gesenkt und zusätzlich aufgeweitet. Die Additive eignen sich auch besonders für Mikroemulsionen mit hydrophoben nichtionischen Tensiden, wie beispielsweise niederethoxylierte Alkohole. Diese Tenside bilden Mikroemulsionen bei sehr tiefen Temperaturen aus. Durch Zugabe des Additivs wird der Temperaturbereich des Einphasengebiets zu höheren Temperaturen verschoben und zusätzlich aufgeweitet. Sowohl für die ionischen als auch die hydrophoben nichtionischen Tenside kann der Temperaturbereich des Einphasengebiets so verschoben werden, dass er für Anwendungen interessanter ist.

Beispiele:

Die für die Beispiele verwendeten polymeren Additive $C_8E_{90}$), $C_{12}E_{90}$, $C_{12}E_{190}$, $C_{12}E_{480}$, $C_{18}E_{80}$, $C_{18}E_{180}$ $C_{12}(E_{90})_2$ wurden durch Ethoxylierung der zugrunde liegenden Alkohole hergestellt. Als Alkohole wurden verwendet: 1-Octanol für $C_8E_{90}$; 1-Dodecanol für $C_{12}E_{90}$, $C_{12}E_{190}$ und $C_{12}E_{480}$; 1-Octadecanol für $C_{18}E_{80}$ und $C_{18}E_{180}$; 1,2-Dodecandiol für $C_{12}(E_{90})_2$. Bei allen Alkoholen handelt es sich um lineare, unverzweigte Alkohole.

Die polymeren Additive $C_8E_{90}$, $C_{12}E_{90}$, $C_{12}E_{190}$, $C_{12}E_{480}$, $C_{18}E_{80}$, $C_{18}E_{180}$ $C_{12}(E_{90})_2$ wurden mittels Gelpermeationschromatographie (GPC) charakterisiert. Zahlenmittlere Molekulargewichte Mn sowie Molekulargewichtsverteilungen Mw/Mn der polymeren Additive wurden anhand einer Kalibrierkurve berechnet, die mittels Polyethylenglycolstandards erhalten wurde.

Die gemessenen Werte für Mw/Mn waren alle kleiner 1,1. Die folgende Tabelle gibt die gemessenen Molekulargewichte wieder. Außerdem sind die aus den Summenformeln der Alkohole berechneten Molekulargewichte der hydrophoben Einheiten (M(hydrophob)) sowie die zahlenmittleren Molekulargewichte der wasserlöslichen Einheiten (Mn(wasserlöslich)), die aus der Differenz von Mn(GPC) und M (hydrophob) berechnet wurden, wiedergegeben. Die Ethoxylierungsgrade wurden aus (Mn(wasserlöslich) durch Division durch 44 (Molekulargewicht einer Ethylenoxideinheit) erhalten.

Tabelle 1:

|  | Mn(GPC) | M(hydrophob) | Mn(wasserlöslich) | Ethoxylierungsgrad |
|---|---|---|---|---|
| $C_8E_{90}$ | 4130 | 113 | 4020 | 91 |
| $C_{12}E_{90}$ | 4270 | 169 | 4100 | 93 |
| $C_{12}E_{190}$ | 8490 | 169 | 8320 | 189 |

(fortgesetzt)

|  | Mn(GPC) | M(hydrophob) | Mn(wasserlöslich) | Ethoxylierungsgrad |
|---|---|---|---|---|
| $C_{12}E_{480}$ | 21200 | 169 | 21000 | 477 |
| $C_{18}E_{80}$ | 3880 | 253 | 3630 | 82 |
| $C_{18}E_{180}$ | 8020 | 253 | 7770 | 176 |
| $C_{12}(E_{90})_2$ | 8180 | 169 | 8010 | 91x2 |

Außerdem wurden folgende weitere polymere Additive verwendet:

Berol EP 35 (Akzo Nobel Surface Chemistry AB). Hierbei handelt es sich um ein C8-Alkohol-Ethoxylat mit durchschnittlich 35 Ethylenoxideinheiten pro Molekül (Herstellerangaben). Die aus der chemischen Struktur berechneten mittleren Molekulargewichte betragen für die hydrophobe Einheit 113 g/mol und für die wasserlösliche Einheit 1560 g/mol.

Tergitol 15-S-30 (Union Carbide). Hierbei handelt es sich um ein Cll-15-Alkohol-Ethoxylat mit durchschnittlich 30 Ethylenoxideinheiten pro Molekül (Handbook of Industrial Surfactants, 2. Auflage, Gower Publishing, ISBN 0-566-07892-9). Die aus der chemischen Struktur berechneten mittleren Molekulargewichte betragen für die hydrophobe Einheit 180 g/mol und für die wasserlösliche Einheit 1340 g/mol.

Brij 700 (Uniqema). Hierbei handelt es sich um ein Stearylalkohol-Ethoxylat mit durchschnittlich 100 Ethylenoxideinheiten pro Molekül (Herstellerangaben). Die aus der chemischen Struktur berechneten mittleren Molekulargewichte betragen für die hydrophobe Einheit 250 g/mol und für die wasserlösliche Einheit 4400 g/mol.

Igepal DM 970 (Rhône-Poulenc). Hierbei handelt es sich um ein Dinonylphenol-Ethoxylat mit durchschnittlich 150 Ethylenoxideinheiten pro Molekül (Handbook of Industrial Surfactants, 2. Auflage, Gower Publishing, ISBN 0-566-07892-9). Die aus der chemischen Struktur berechneten mittleren Molekulargewichte betragen für die hydrophobe Einheit 330 g/mol und für die wasserlösliche Einheit 6600 g/mol.

Bei allen hier beschriebenen polymeren Additiven bis auf $C_{12}(E_{90})_2$ handelt es sich um den Strukturtyp einer linearen wasserlöslichen Polymerkette, die an einem Ende mit einer hydrophoben Einheit versehen ist. Bei dem poly-meren Additiv $C_{12}(E_{90})_2$ handelt es sich um den Strukturtyp einer linearen wasserlöslichen Polymerkette, die in der Kettenmitte mit einer hydrophoben Einheit versehen ist.

Für die Beispiele wurden folgende Tenside und Öle verwendet. Tetraethylenglycolmonodecylether ($C_{10}E_4$); IMBENTIN AG 100/040, C10 Alkoholethoxylat mit durchschnittlich 4 Ethylenoxideinheiten (Herstellerangabe)(Kolb, Schweiz); Hoesch T5 Isotridecanolat mit durchschnittlich 5 Ethylenoxideinheiten (Julius Hoesch GmbH & Co. KG, Düren); Natrium bis(2-ethylhexyl) sulfosuccinat (AOT); Hydroxy(polyethylenoxy)propylterminertes polydimethyl-Siloxan Molekulargewicht 550 - 650 g/mol 50 % ($CH_2$-$CH_2$-O) (MCR-C13,Gelest Inc. Morrisville, PA, USA) ($C_4D_3E_8$); n- Dekan; Octamethyltrisiloxan (MDM); Hydroseal G232H, Gemisch aus $C_{13}$-$C_{15}$ aliphatischen Kohlenwasserstoffen, Siedebereich 235-265 °C, Flammpunkt 102 °C (Julius Hoesch GmbH & Co. KG, Düren); Rapsmethylester (Biodiesel/ADM-Oelmühle Hamburg AG/ADM-Oelmühle Leer Connemann GmbH + Co. KG).

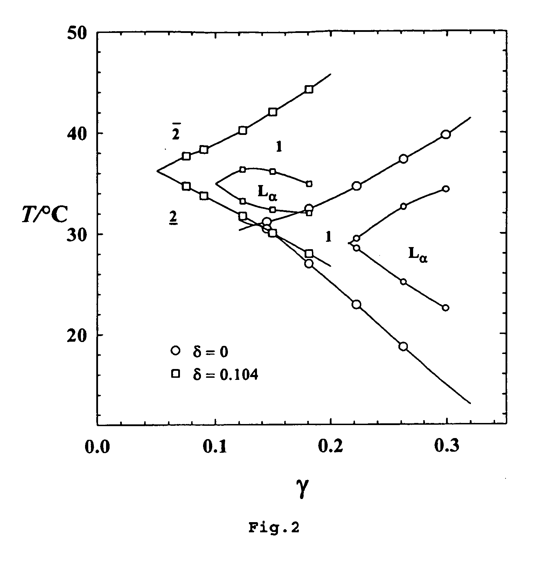

Die in den Figuren 2-17 dargestellten Temperatur-Konzentrations- (T/y) Phasendiagramme beziehen sich, wenn nicht anders beschrieben, auf Systeme mit konstantem Wasser/Öl Volumenverhältnis von 1:1 ($\phi$ = 0.5) und sollen im Folgenden erläutert werden.

Im Folgenden werden einige Begriffe eingeführt:

C = Ein beliebiges Tensid oder Emulgator, wie anionisches, kationisches, nichtionisches Tensid oder Zuckertensid, sowie Gemische, die mindestens zwei Tenside enthalten.

D = Additiv, welches dem Tensid C erfindungsgemäß zugefügt wird.

$\gamma$ = Gesamttensidkonzentration (Massenbruch) aus C und D mit

$$\gamma = \frac{m(C) + m(D)}{m_{ges}}$$

Hierin sind:

m = Masse in g.

$\gamma$ = dimensionsloser Massenbruch

$m_{ges}$ = Gesamtmasse aus $m_{wasser}$ + $m_{Öl}$ + m (C) + m(D)

$\delta$ = Massenbruch des Additivs D im Gemisch Tensid C + Additiv D, entspricht $\quad \delta = \dfrac{m(D)}{m(C)+m(D)}$

mit m = Masse in g und

$\delta$ = Massenbruch (dimensionslos)

In den Diagrammen sind die Kurven zu jeweils einem $\delta$ - Wert eingezeichnet, welche die Begrenzung des jeweiligen zu einem $\delta$ - Wert gehörigen Einphasengebiets charakterisieren. Die Spitze der jeweiligen Kurve ist der Fischschwanzpunkt. Die Bezeichnung 1 kennzeichnet dabei die Bereiche einphasiger Mikroemulsion, $\underline{2}$ beschreibt eine Öl in Wasser - Mikroemulsion in Koexistenz mit einer Ölphase und $\overline{2}$ beschreibt eine Wasser in Öl - Mikroemulsion in Koexistenz mit einer Wasserphase. Lamellare Phasen werden in den Abbildungen mit $L_\alpha$ gekennzeichnet, fehlen diese, so treten im untersuchten Bereich keine lamellaren Phasen auf. Die folgenden Phasendiagramme geben jeweils die Ausdehnung der Mikroemulsionsphase in Abhängigkeit von der Tensidkonzentration y und der Temperatur T wieder.

Figur 2 zeigt wie sich die Effizienz der einphasigen Mikroemulsion im System Wasser - n-Dekan - $C_{10}E_4$ bei Zugabe des Polymers $C_{12}E_{90}$ vergrößert. Ersetzt man 10 % des Tensides durch $C_{12}E_{90}$ ($\delta$ = 0.10) , so verschiebt sich der Fischschwanzpunkt von $\gamma$=0.13 nach $\gamma$=0.05, d. h. der zur Solubilisierung notwendige Tensidbedarf wird mehr als halbiert. Die Breite des Temperaturfensters der Mikroemulsionsphase erhöht sich deutlich und durch die Polymerzugabe verschiebt sich die Temperatur des Fischschwanzpunktes nur geringfügig zu höherer Temperatur. Insgesamt wird die Temperaturlage des Fischschwanzpunktes um etwa 5-7 °C erhöht.

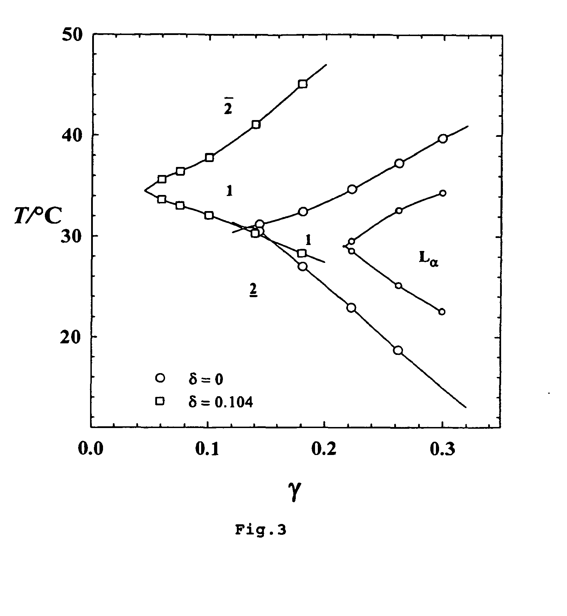

Die Effizienz des Gesamttensides wird auch in dem in Figur 3 gezeigten System Wasser - n-Dekan - $C_{10}E_4$ bei Zugabe des polymeren Additivs $C_{12}E_{190}$ vergrößert. Die Effizienzsteigerung und die Ausdehnung der einphasigen Mikroemulsion ist dabei leicht besser als in dem in Figur 2 gezeigten System. Durch die Wahl des größeren Polymers $C_{12}E_{190}$ wird jedoch die Bildung der lamellaren Phase im untersuchten Bereich wirkungsvoll unterdrückt.

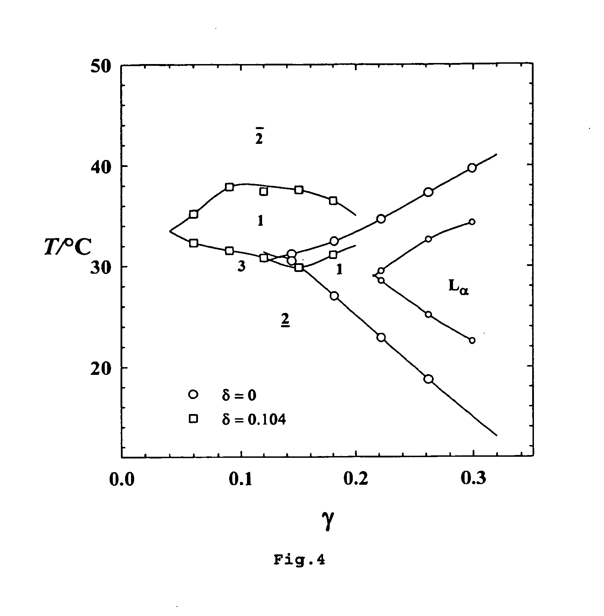

Figur 4 zeigt die Effizienzsteigerung des polymeren Additivs $C_{12}E_{480}$ im System Wasser - n-Dekan - $C_{10}E_4$. In diesem Beispiel ist die Effizienzsteigerung hinsichtlich der Lage des Fischschwanzpunktes leicht besser als in dem in Figur 2 und Figur 3 gezeigten System Wasser - n-Dekan - $C_{10}E_4$ - $C_{12}E_{90}$ bzw. Wasser - n-Dekan - $C_{10}E_4$ - $C_{12}E_{190}$. Allerdings ist die Aufweitung des Temperaturfensters bei $\gamma \geq 0.10$ weniger ausgeprägt als in den in Figur 2 und Figur 3 gezeigten Systemen.

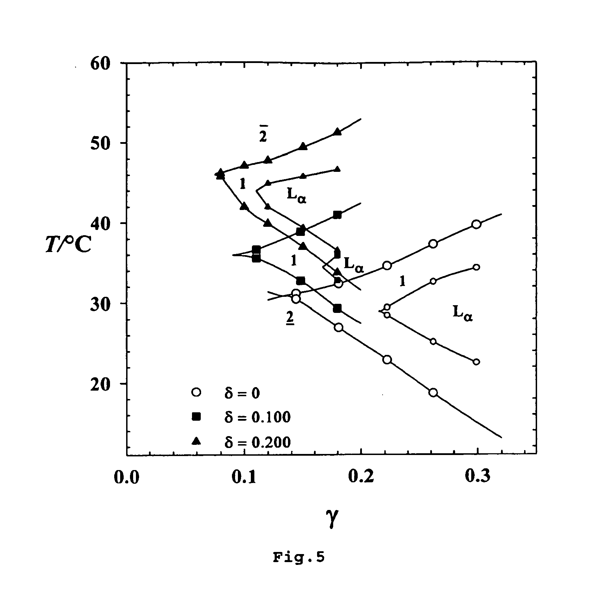

Es lassen sich aber auch Polymere mit kleineren hydrophilen Blöcken einsetzen, dies wird in Figur 5 am System Wasser - n-Dekan - $C_{10}E_4$ - Tergitol 15S30 gezeigt. In diesem Beispiel wurde das polymerfreie System ($\delta$=0) mit dem System mit 10 % polymeres Additiv ($\delta$ = 0.100) sowie 20 % polymeres Additiv ($\delta$=0.200) in der Tensidmischung verglichen. Die Effizienzsteigerung ist zwar geringer als in den vorangegangenen Beispielen, dennoch erhält man vor allem bei $\delta$ =0.200 eine deutliche Aufweitung des Mikroemulsionsbereiches. Verbunden ist dies allerdings mit einer Erhöhung des Temperaturbereichs der Mikroemulsion um ca. 15 °C im Vergleich zum polymerfreien System. Während die lamellare Phase relativ zum Fischschwanzpunkt etwa konstant bleibt, für $\delta$=0.100 verglichen mit dem polymerfreien System, ist für $\delta$ = 0.200 die lamellare Phase deutlich stärker ausgeprägt.

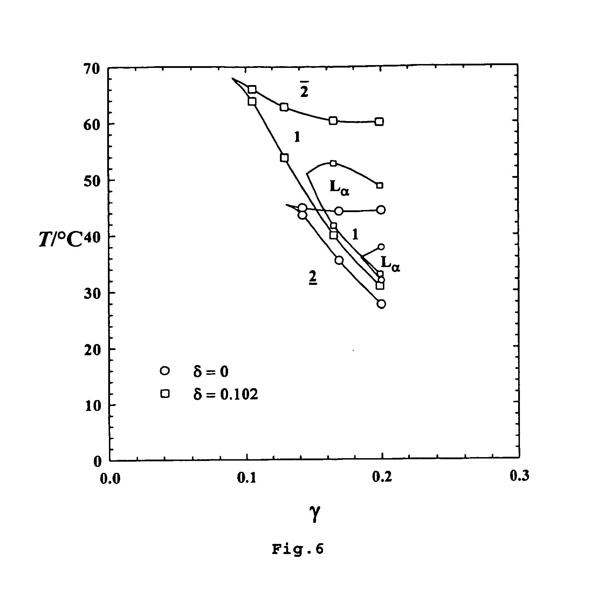

In den Figuren 6-8 wird anstatt eines $C_{12}$ Restes ein hydrophoberer $C_{18}$ Kohlenwasserstoffrest benutzt.

Figur 6 zeigt das System Wasser - Hydroseal G232H - IMBENTIN AG 100/04 0 - Brij 700 bei $\delta$ = 0 und $\delta$ = 0.102 . Der Fischschwanzpunkt verschiebt sich dabei von $\gamma$=0.12 nach y=0.08. Das Temperaturfenster wird stark aufgeweitet und die Phaseninversionstemperatur von T = 44°C auf T = 68°C erhöht. Diese Temperaturerhöhung kann auch genutzt werden um hydrophobere, aber effiziente Tenside in den gewünschten Temperaturbereich zu bekommen. So haben weniger ethoxylierte Tenside einen bei tieferen Temperaturen liegenden Mikroemulsionsbereich als vergleichbare höher ethoxylierte Tenside, die die gleiche hydrophobe Gruppe aufweisen. Außerdem sind nieder ethoxylierte Tenside leicht effizienter als höher ethoxylierte. Deshalb kann es Sinn machen, ein für den gewünschten Temperaturbereich zu nieder ethoxyliertes Tensid zu verwenden und mit Hilfe des polymeren Additivs den Temperaturbereich zu erhöhen.

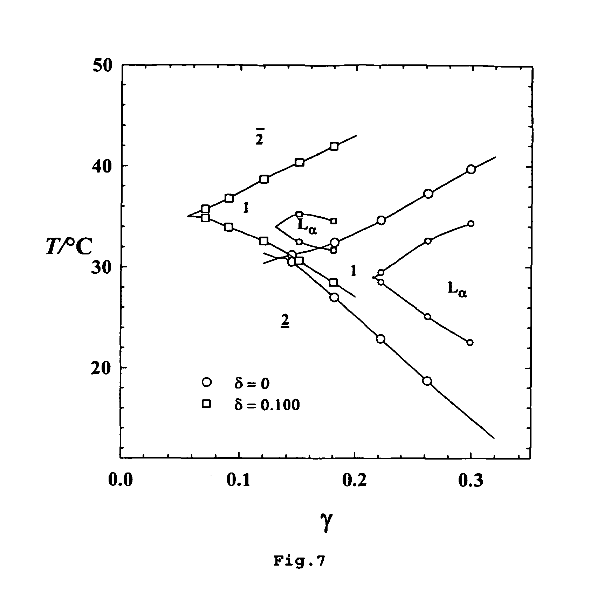

Figur 7 zeigt den Einfluss eines größeren hydrophoberen Restes im Polymer $C_{18}E_{80}$ auf das Phasenverhalten des Systems Wasser - n-Dekan - $C_{10}E_4$. Das Phasenverhalten und die Effizienzsteigerung ist dabei vergleichbar mit dem in Figur 1 gezeigten Wasser - n-Dekan - $C_{10}E_4$ - $C_{12}E_{80}$. Verbunden mit der Effizienzsteigerung ist auch in diesem Beispiel die Bildung einer niederviskosen lamellaren Phase, die relativ zum Fischschwanzpunkt noch geringer ausgeprägt ist als beim System ohne Polymer. Zu erwarten wäre auch hier, dass aufgrund der Effizienzsteigerung nahezu der gesamte Mikroemulsionsbereich mit lamellarer Phase ausgefüllt ist.

Die vollständige Unterdrückung der lamellaren Phase wird im System Wasser - n-Dekan - $C_{10}E_4$ - $C_{18}E_{180}$ in

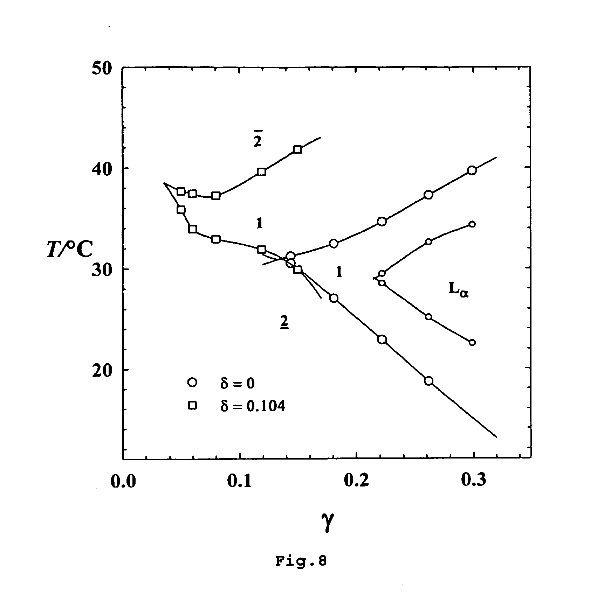

Figur 8 erreicht. Der Fischschwanzpunkt liegt im polymerhaltigen System ($\delta$=0.104) bei $\gamma$=0.03 und ist damit um etwa um den Faktor 4 kleiner als im polymerfreien System ($\delta$=0).

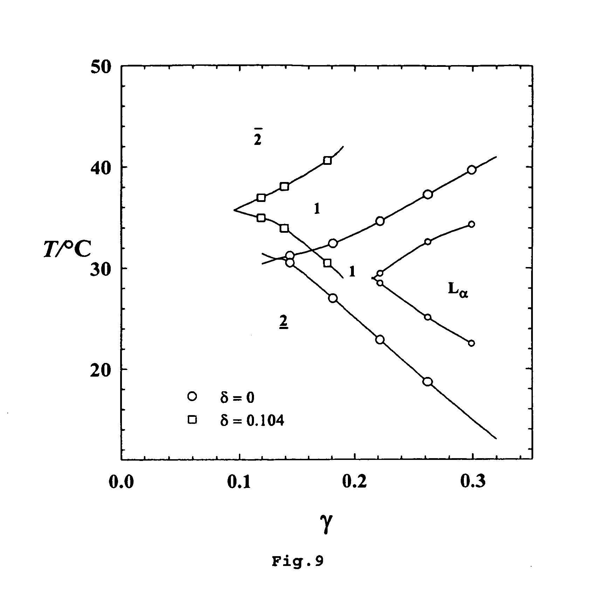

Auch kleinere hydrophobe Reste als $C_{12}$ können als hydrophobe Einheiten in den erfindungsgemäßen polymeren Additiven verwendet werden. Dies wird in Figur 9 gezeigt. Die Effizienz des Systems Wasser - n-Dekan - $C_{10}E_4$ wird bei Zugabe von 10 % ($\delta$=0.104) Berol EP35 gesteigert, ohne die dabei im effizienten Tensidsystem häufige lamellare Phase auszubilden. Auch das Temperaturfenster wird aufgeweitet.

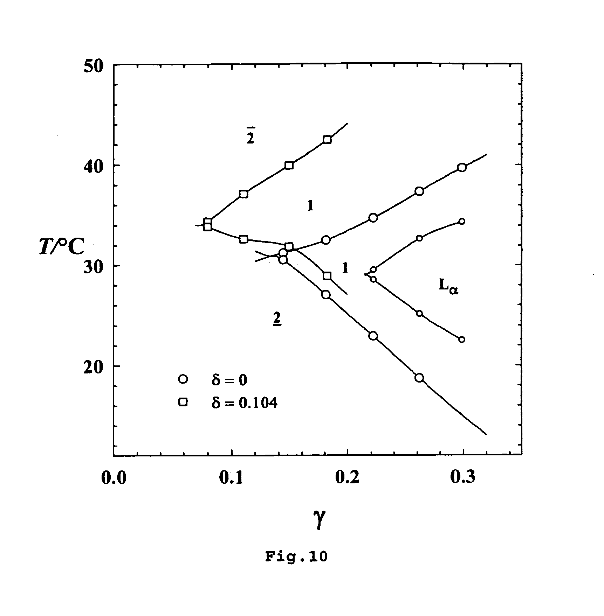

Figur 10 zeigt die Effizienzsteigerung im System Wasser - n-Dekan - $C_{10}E_4$ bei Zugabe des Polymers $C_8E_{90}$. Ersetzt man 10 % des Tensides durch $C_8E_{90}$ so halbiert sich der Tensidgesamtbedarf von $\gamma = 0.13$ auf $\gamma = 0.07$. Die Temperaturausdehnung der einphasigen Mikroemulsion nimmt dabei im gleichen Maße zu, eine lamellare Phase fehlt im untersuchten System.

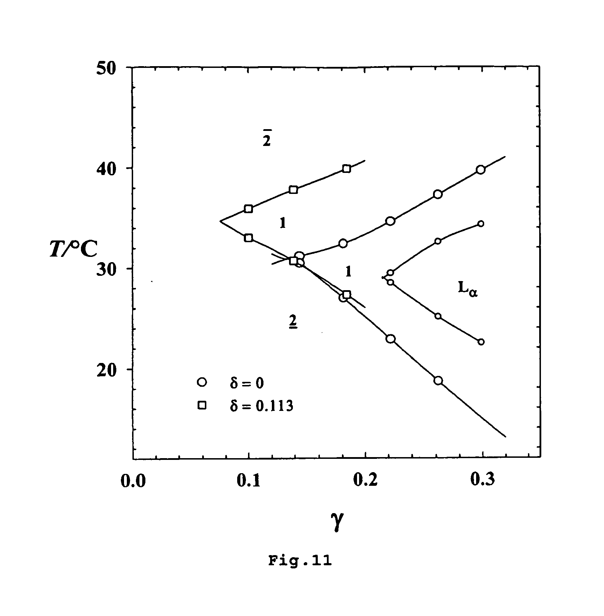

Figur 11 zeigt, wie sich die Effizienz des Gesamttensides mit der Zugabe des Polymers IGEPAL DM970 in der Mischung aus Wasser - n-Dekan - $C_{10}E_4$ bei $\delta = 0.113$ vergrößert. Lamellare Phasen fehlen dabei in der Mikroemulsion mit Polymerzusatz. Auch in diesem System wird die Phaseninversionstemperatur um etwa 5 °C zu höheren Temperaturen verschoben. Wie in den vorangegangenen Beispielen wurde ein konstantes Wasser/Öl Verhältnis von $\phi = 0.5$ verwendet.

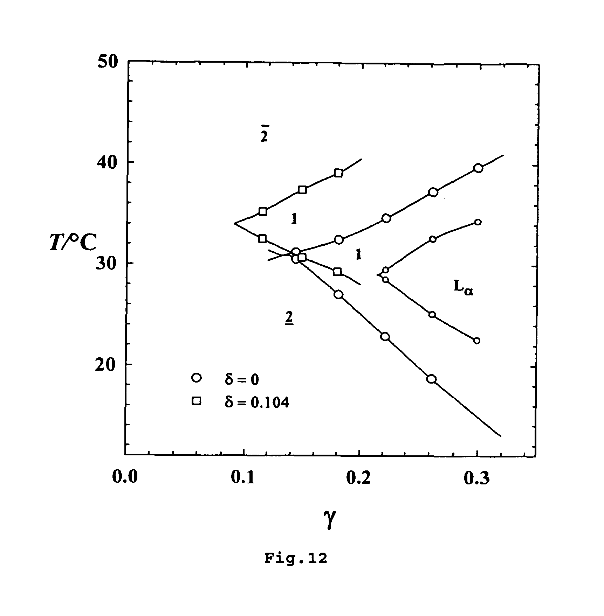

Figur 12 zeigt, wie sich die zur Bildung notwendige Gesamttensidmenge mit der Zugabe des Polymers $C_{12}$($E_{90}$)$_2$ in der Mischung aus Wasser - n-Dekan - $C_{10}E_4$ bei $\delta = 0.104$ verringert. Die Phaseninversionstemperatur verschiebt sich dabei nur geringfügig um etwa 4°C zu höherer Temperatur, das grundsätzliche Phasenverhalten bleibt durch die Polymerzugabe unverändert. Eine lamellare Phase tritt im untersuchten Bereich nicht auf.

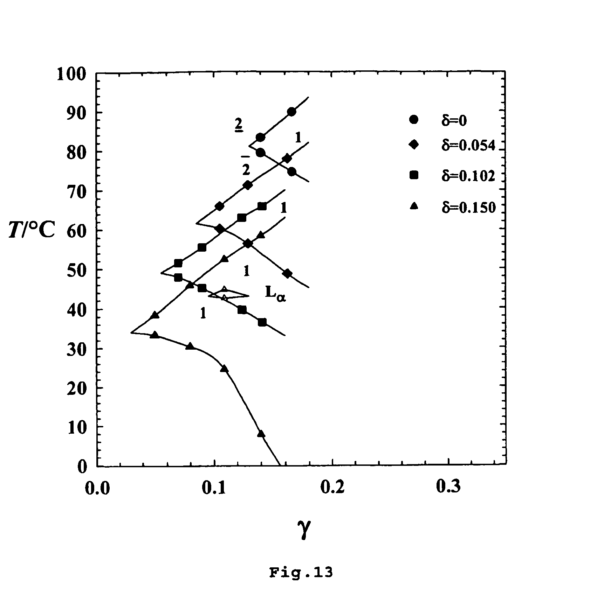

Eine noch deutlichere Aufweitung des Temperaturfensters und Steigerung der Effizienz kann man beim Einsatz des erfindungsgemäßen polymeren Additivs mit dem ionischen Tensid AOT beobachten. Figur 13 zeigt dies im System Wasser/NaC1(1%) - n-Dekan - AOT - Brij 700 in Abhängigkeit von verschiedenen Polymerkonzentrationen $\delta$. Dabei wird mit steigender Polymerzugabe $\delta$ die Phaseninversionstemperatur so weit abgesenkt, dass sie einen für die meisten Anwendungen geeigneten Bereich ermöglicht, wohin gegen ohne polymere Additive Mikroemulsionen im wenig interessanten Temperaturbereich auftreten. Gleichzeitig sinkt mit steigendem Polymeranteil der notwendige minimale Tensidbedarf und die Breite der einphasigen Mikroemulsion wird deutlich vergrößert. Selbst bei $\delta = 0.15$ ist nur eine minimale lamellare Insel vorhanden.

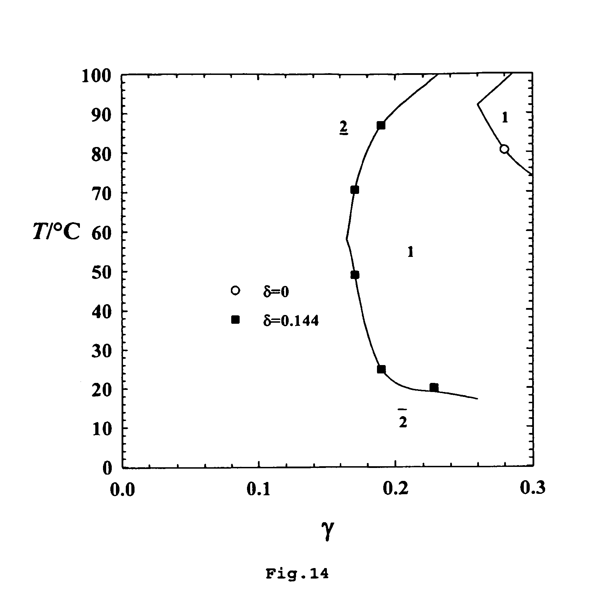

Die neue Polymerklasse eignet sich auch sehr gut für Mikroemulsionen mit polaren Ölen wie Esterölen. Dies wird in Figur 14 am System Wasser/NaC1(1%) - Rapsmethylester - AOT - Brij 700 gezeigt. Im polymerfreien System ($\delta$ = 0) konnte nur ein Messpunkt bestimmt werden, der tatsächliche Mikroemulsionsbereich entspricht aber etwa dem eingezeichneten Bereich und liegt bei etwa 90°C. Selbst bei einem hohen Tensidanteil von 28 % ist die untere Phasengrenze bei 80 °C. Ersetzt man 14 % des Tensides durch Brij 700 ($\delta$ = 0.144), so erhält man einen breiten Mikroemulsionsbereich zwischen 20 °C und 80 °C bei einem Tensidanteil kleiner 20 %. Der Einsatz von Rapssäuremethylester als Ölkomponente ist damit bei kleinen Tensidkonzentrationen nur mit Hilfe des Polymers Brij 700 möglich. Wird statt des erfindungsgemäßen polymeren Additivs ein nichtionisches Tensid wie Hoesch T5 in der gleichen Konzentration verwendet, so bildet sich keine einphasige Mikroemulsionsphase.

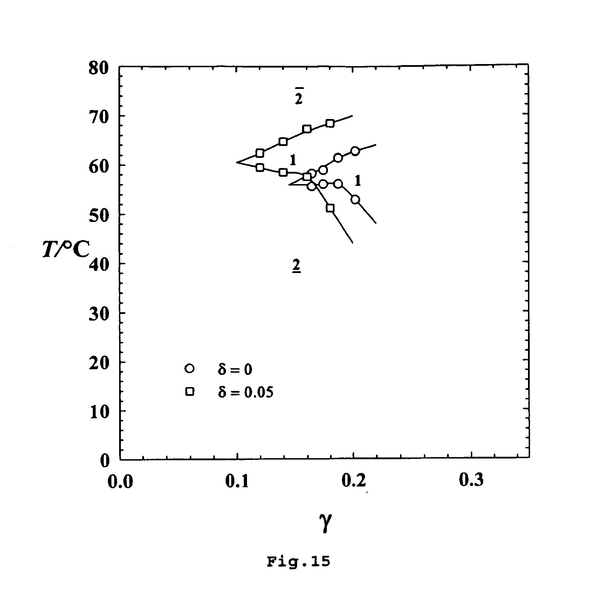

Auch Silikonöle eignen sich als Ölkomponente von Mikroemulsionen. Figur 15 zeigt wie sich die Effizienz des Gesamttensides und das Temperaturfenster mit der Zugabe des Polymers Brij 700 in der Mischung aus Wasser - MDM - $C_4D_3E_8$ bei $\delta = 0.05$ vergrößert. Der minimale Tensidbedarf verschiebt sich im System mit Brij 700 in der Tensidmischung ($\delta$ = 0.05) von $\gamma = 0.14$ nach $\gamma = 0.10$. Der notwendige Gesamttensidanteil wird dabei um etwa 30 % reduziert. Eine lamellare Phase tritt im untersuchten System nicht auf.

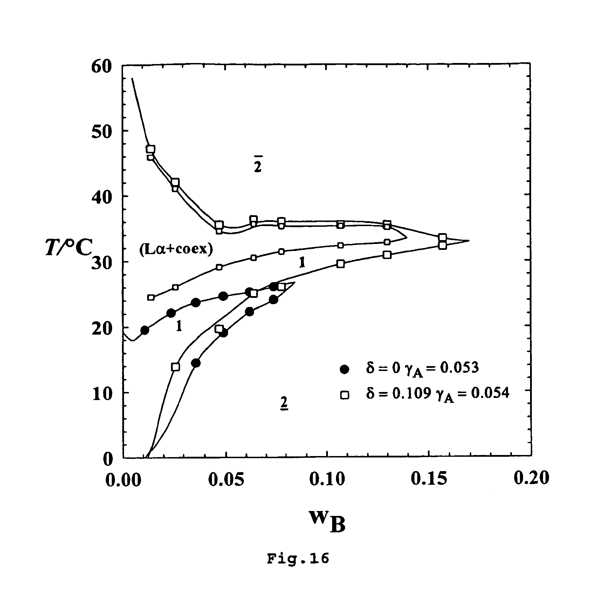

Die Effizienz steigernde Wirkung der neuen Polymerklasse lässt sich auch in Systemen mit geringen Ölanteilen beobachten. Figur 16 zeigt ein o/w Mikroemulsion in der Mischung aus Wasser - n-Dekan - $C_{10}E_4$ - Brij 700. Das konstante Tensid/Wasser Verhältnis $Y_A$ beträgt 0.053, der Ölanteil $W_B$ in der Gesamtmischung wird in dieser Auftragung als Funktion der Temperatur variiert. Im polymerhaltigen System ($\delta$ = 0.109) können dabei deutlich größere Anteile Öl solubilisiert werden als im polymerfreien System. Der Massenbruch des maximalen Ölanteils $W_B$ wird dabei von $W_B$ = 0.07 auf $W_B$ = 0.18 gesteigert. Neben der Effizienzsteigerung lässt sich auch eine deutliche Aufweitung des Temperaturfensters beobachten. Im polymerhaltigen System ($\delta$ = 0.109) tritt eine lamellare Phase auf, die in ihrer Ausdehnung jedoch geringer ist als aufgrund der Effizienz zu erwarten wäre.

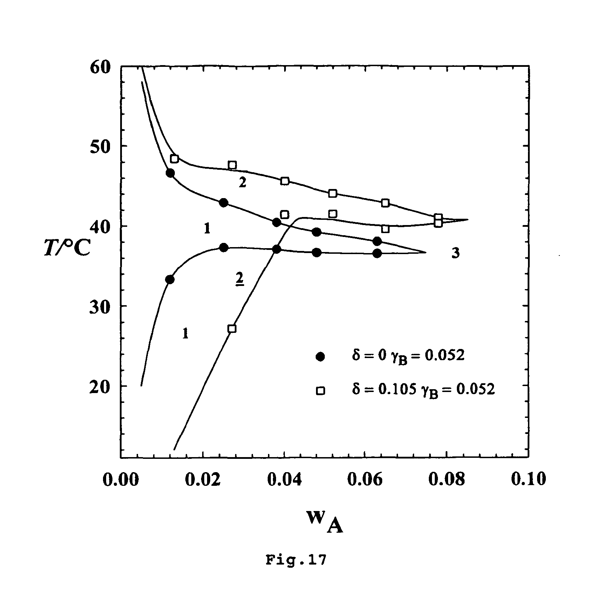

Figur 17 zeigt die Effizienz steigernde Wirkung der neuen Polymerklasse in w/o Mikroemulsionen mit kleinen Wasseranteilen. Das konstante Tensid/Öl Verhältnis $Y_B$ beträgt 0.052, der Wasseranteil $W_A$ in der Gesamtmischung aus Wasser - n-Dekan - $C_{10}E_4$ - Brij 700 wird in dieser Auftragung als Funktion der Temperatur variiert. Ersetzt man 10 % des Tensides durch Brij 700, so wird das Temperaturfenster der einphasigen Mikroemulsion deutlich vergrößert und die Effizienz des Systems gesteigert. Im untersuchten System treten keine lamellare Phasen auf.

**Patentansprüche**

1. Verfahren zur Aufweitung des Temperaturfensters von Mikroemulsionen, bei dem der Mikroemulsion ein polymeres Additiv mit mindestens einer hydrophoben Einheit und mindestens einer wasserlöslichen Einheit zugefügt wird, **dadurch gekennzeichnet,** **dass** das Verhältnis der zahlenmittleren Molekulargewichte von wasserlöslichen zu hydrophoben Einheiten des Additivs 2 bis 200 beträgt, wobei jede hydrophobe Einheit ein Molekulargewicht von maximal 500 g/mol besitzt und dass die Additive Polymere sind, welche aus einer linearen wasserlöslichen Einheit bestehen, die mindestens an einem Kettenende mindestens eine hydrophobe Einheit besitzt.

2. Verfahren zur Aufweitung des Temperaturfensters von Mikroemulsionen, bei dem der Mikroemulsion ein polymeres Additiv mit mindestens einer hydrophoben Einheit und mindestens einer wasserlöslichen Einheit zugefügt wird, **dadurch gekennzeichnet,** **dass** das Verhältnis der zahlenmittleren Molekulargewichte von wasserlöslichen zu hydrophoben Einheiten des Additivs 2 bis 200 beträgt, wobei jede hydrophobe Einheit ein Molekulargewicht von maximal 1000 g/mol besitzt und dass die Additive Polymere sind, welche aus einer linearen wasserlöslichen Einheit bestehen, die eine hydrophobe Einheit als nicht-terminalen Substituenten besitzt oder dass das Additiv ein Polymer ist, bei dem eine hydrophobe Einheit an einer Stelle zwischen wasserlöslichen Einheiten des Polymers eingegliedert ist oder dass das polymere Additiv aus einem verzweigten wasserlöslichen Teil besteht, der an einem Ende eine hydrophobe Einheit trägt.

3. Verfahren nach Anspruch 1 oder 2, **dadurch gekennzeichnet,** **dass** das Verhältnis der zahlenmittleren Molekulargewichte von wasserlöslichen zu hydrophoben Einheiten des Additivs 5 bis 200 beträgt.

4. Verfahren nach Anspruch 3, **dadurch gekennzeichnet,** **dass** das Verhältnis der zahlenmittleren Molekulargewichte von wasserlöslichen zu hydrophoben Einheiten des Additivs 10 bis 50 beträgt.

5. Verfahren nach Anspruch 1 bis 4, **dadurch gekennzeichnet,** **dass** das zahlenmittlere Molekulargewicht jeder wasserlöslichen Einheit des Additivs zwischen 500 und 100000 g/mol liegt.

6. Verfahren nach Anspruch 5, **dadurch gekennzeichnet,** **dass** das zahlenmittlere Molekulargewicht jeder wasserlöslichen Einheit des Additivs zwischen 2000 und 20000 g/mol liegt.

7. Verfahren nach Anspruch 6, **dadurch gekennzeichnet,** **dass** das zahlenmittlere Molekulargewicht jeder wasserlöslichen Einheit des Additivs zwischen 3000 und 10000 g/mol liegt

8. Verfahren nach einem der Ansprüche 2 bis 7, **dadurch gekennzeichnet,** **dass** das zahlenmittlere Molekulargewicht jeder hydrophoben Einheit des Additivs zwischen 80 und 1000 g/mol liegt.

9. Verfahren nach Anspruch 1-8, **dadurch gekennzeichnet,** **dass** das zahlenmittlere Molekulargewicht jeder hydrophoben Einheit des Additivs zwischen 110 und 500 g/mol liegt.

10. Verfahren nach Anspruch 9, **dadurch gekennzeichnet,** **dass** das zahlenmittlere Molekulargewicht jeder hydrophoben Einheit des Additivs zwischen 110 und 280 g/mol liegt.

11. Verfahren nach einem der Ansprüche 1 bis 10, **dadurch gekennzeichnet,** **dass** die wasserlösliche Einheit des Additivs nichtionisch ist.

**12.** Verfahren nach einem der Ansprüche 1 bis 10,
**dadurch gekennzeichnet,**
**dass** die wasserlösliche Einheit des Additivs ionisch ist.

**13.** Verfahren nach einem der Ansprüche 1 bis 10,
**dadurch gekennzeichnet,**
**dass** sich die wasserlösliche Einheit des Additivs aus einem ionischen und nichtionischen Bestandteil zusammensetzt.

**14.** Verfahren nach einem der Ansprüche 1 bis 13,
**dadurch gekennzeichnet,**
**dass** der wasserlösliche Bestandteil des Additivs eine Komponente aus der Gruppe bestehend aus Polyethylenoxid, Polyethylenglycol, Copolymerisate aus Ethylenoxid und Propylenoxid, Polyacrolein Polyvinylalkohol und dessen wasserlösliche Derivate, Polyvinylpyrrolidon, Polyvinylpyridin, Polymethacrylsäure, Polymaleinsäureanhydrid, Polyacrylsäure, Polystyolsulfonsäure und deren wasserlösliche Salze ist.

**15.** Verfahren nach einem der Ansprüche 1 bis 14,
**dadurch gekennzeichnet,**
**dass** die hydrophoben Einheiten des Additivs Alkylreste sind.

**16.** Verfahren nach Anspruch 2 bis 15,
**dadurch gekennzeichnet,**
**dass** der Akylrest 6 bis 50 Kohlenstoffatome umfasst.

**17.** Verfahren nach Anspruch 16,
**dadurch gekennzeichnet,**
**dass** der Akylrest 8 bis 20 Kohlenstoffatome umfasst.

**18.** Verfahren nach einem der Ansprüche 1 bis 10,11, 14 bis 17,
**dadurch gekennzeichnet,**
**dass** das Additiv ein Alkoholethoxylat bestehend aus einem einwertigem Alkohol mit 8-20 C-Atomen und 25-500 Ethylenoxideinheiten ist.

**19.** Verfahren nach Anspruch 1-18,
**dadurch gekennzeichnet,**
**dass** das Verhältnis der Molekulargewichte von wasserlöslichem zu hydrophobem Teil 10 bis 50 beträgt.

**Claims**

**1.** Method for expanding the temperature window of microemulsions, wherein a polymer additive having at least one hydrophobic unit and at least one water-soluble unit is added to the microemulsion, **characterised in that** the ratio of the number-average molecular weights of water-soluble to hydrophobic units in the additive is in the range 2 to 200, wherein each hydrophobic unit has a molecular weight of not more than 500 g/mol and the additives are polymers comprising a linear water-soluble unit which has at least one hydrophobic unit at least at one end of the chain.

**2.** Method for expanding the temperature window of microemulsions, wherein a polymer additive having at least one hydrophobic unit and at least one water-soluble unit is added to the microemulsion, **characterised in that** the ratio of the number-average molecular weights of water-soluble to hydrophobic units in the additive is in the range 2 to 200, wherein each hydrophobic unit has a molecular weight of not more than 1000 g/mol and the additives are polymers comprising a linear water-soluble unit which has a hydrophobic unit as a non-terminal substituent or that the additive is a polymer wherein a hydrophobic unit is integrated at a site between water-soluble units of the polymer or that the polymer additive comprises a branched water-soluble part which carries a hydrophobic unit at one end thereof.

**3.** Method according to claim 1 or 2, **characterised in that** the ratio of number-average molecular weights of water-soluble to hydrophobic units of the additive is in the range 5 to 200.

4. Method according to claim 3, **characterised in that** the ratio of number-average molecular weights of water-soluble to hydrophobic units of the additive is in the range 10 to 50.

5. Method according to claim 1 to 4, **characterised in that** the number-average molecular weight of each water-soluble unit of the additive is in the range 500 to 100000 g/mol.

6. Method according to claim 5, **characterised in that** the number-average molecular weight of each water-soluble unit of the additive is in the range 2000 to 20000 g/mol.

7. Method according to claim 6, **characterised in that** the number-average molecular weight of each water-soluble unit of the additive is in the range 3000 to 10000 g/mol.

8. Method according to one of the claims 2 to 7, **characterised in that** the number-average molecular weight of each hydrophobic unit of the additive is in the range 80 to 1000 g/mol.

9. Method according to claim 1-8, **characterised in that** the number-average molecular weight of each hydrophobic unit of the additive is in the range 110 to 500 g/mol.

10. Method according to claim 9, **characterised in that** the number-average molecular weight of each hydrophobic unit of the additive is in the range 110 to 280 g/mol.

11. Method according to one of the claims 1 to 10, **characterised in that** the water-soluble unit of the additive is non-ionic.

12. Method according to one of the claims 1 to 10, **characterised in that** the water-soluble unit of the additive is ionic.

13. Method according to one of the claims 1 to 10, **characterised in that** the water-soluble unit of the additive comprises an ionic and a non-ionic constituent combined.

14. Method according to one of the claims 1 to 13, **characterised in that** the water-soluble constituent of the additive is a component belonging to the group consisting of polyethylene oxide, polyethylene glycol, copolymers of ethylene oxide and propylene oxide, polyacrolein polyvinyl alcohol and the water-soluble derivatives thereof, polyvinylpyrrolidone, polyvinylpyridine, polymethacrylic acid, polymaleic anhydride, polyacrylic acid, polystyrene sulphonic acid, and the water-soluble salts thereof.

15. Method according to one of the claims 1 to 14, **characterised in that** the hydrophobic units of the additive are alkyl groups.

16. Method according to claim 2 to 15, **characterised in that** the alkyl group comprises in the range 6 to 50 carbon atoms.

17. Method according to claim 16, **characterised in that** the alkyl group comprises in the range 8 to 20 carbon atoms.

18. Method according to one of the claims 1 to 10, 11, 14 to 17, **characterised in that** the additive is an alcohol ethoxylate comprising a monohydroxylic alcohol having in the range 8 to 20 carbon atoms and in the range 25 to 500 ethylene oxide units.

19. Method according to claim 1 to 18, **characterised in that** the ratio of molecular weights of the water-soluble to the hydrophobic portions is in the range 10 to 50.

**Revendications**

1. Procédé d'élargissement de la fenêtre de température de microémulsions, dans lequel on ajoute à la microémulsion un additif polymère comportant au moins un motif hydrophobe et au moins un motif hydrosoluble, **caractérisé en ce que** le rapport des poids moléculaires moyens en nombre des motifs hydrosolubles à hydrophobes de l'additif est de 2 à 200, chaque motif hydrophobe possédant un poids moléculaire au maximum de 500 g/mole et, **en ce que** les additifs sont des polymères qui consistent en un motif hydrosoluble linéaire qui possède au moins à une fin de chaîne, un motif hydrophobe.

**2.** Procédé d'élargissement de la fenêtre de température de microémulsions dans lequel on ajoute à la microémulsion, un additif polymère comportant au moins un motif hydrophobe et au moins un motif hydrosoluble, **caractérisé en ce que** le rapport des poids moléculaires moyens en nombre des motifs hydrosolubles à hydrophobes de l'additif est de 2 à 200, chaque motif hydrophobe possédant un poids moléculaire au maximum de 1 000 g/mole et, **en ce que** les additifs sont des polymères qui consistent en un motif hydrosoluble linéaire qui possède un motif hydrophobe comme substituants non terminaux ou **en ce que** l'additif est un polymère dans lequel un motif hydrophobe est intégré sur un site entre des motifs hydrosolubles du polymère ou **en ce que** l'additif polymère consiste en une partie hydrosoluble ramifiée qui porte à une extrémité, un motif hydrophobe.

**3.** Procédé selon la revendication 1 ou 2, **caractérisé en ce que** le rapport des poids moléculaires moyens en nombre des motifs hydrosolubles à hydrophobes de l'additif est de 5 à 200.

**4.** Procédé selon la revendication 3, **caractérisé en ce que** le rapport des poids moléculaires moyens en nombre des motifs hydrosolubles à hydrophobes de l'additif est de 10 à 50.

**5.** Procédé selon la revendication 1 à 4, **caractérisé en ce que** le poids moléculaire moyen en nombre de chaque motif hydrosoluble de l'additif se situe entre 500 et 100 000 g/mole.

**6.** Procédé selon la revendication 5, **caractérisé en ce que** le poids moléculaire moyen en nombre de chaque motif hydrosoluble de l'additif se situe entre 2 000 et 20 000 g/mole.

**7.** Procédé selon la revendication 6, **caractérisé en ce que** le poids moléculaire moyen en nombre de chaque motif hydrosoluble de l'additif se situe entre 3 000 et 10 000 g/mole.

**8.** Procédé selon l'une des revendications 2 à 7, **caractérisé en ce que** le poids moléculaire moyen en nombre de chaque motif hydrophobe de l'additif se situe entre 80 et 1 000 g/mole.

**9.** Procédé selon les revendications 1 à 8, **caractérisé en ce que** le poids moléculaire moyen en nombre de chaque motif hydrophobe de l'additif se situe entre 110 et 500 g/mole.

**10.** Procédé selon la revendication 9, **caractérisé en ce que** le poids moléculaire moyen en nombre de chaque motif hydrophobe de l'additif se situe entre 110 et 280 g/mole.

**11.** Procédé selon l'une des revendications 1 à 10, **caractérisé en ce que** le motif hydrosoluble de l'additif est non ionique.

**12.** Procédé selon l'une des revendications 1 à 10, **caractérisé en ce que** le motif hydrosoluble de l'additif est ionique.

**13.** Procédé selon l'une des revendications 1 à 10, **caractérisé en ce que** le motif hydrosoluble de l'additif est constitué d'un composant ionique et non ionique.

**14.** Procédé selon l'une des revendications 1 à 13, **caractérisé en ce que**

le composant hydrosoluble de l'additif est un composant du groupe comprenant l'oxyde de polyéthylène, le polyéthylène glycol, les copolymères d'oxyde d'éthylène et d'oxyde de propylène, la polyacroléine, l'alcool polyvinylique et leurs dérivés hydrosolubles, la polyvinylpyrrolidone, la polyvinylpyridine, l'acide polyméthacrylique, l'anhydride d'acide polymaléique, l'acide polyacrylique, l'acide polystyrène-sulfonique et leurs sels hydrosolubles.

15. Procédé selon l'une des revendications 1 à 14,
**caractérisé en ce que**
les motifs hydrophobes de l'additif sont des radicaux alkyle.

16. Procédé selon les revendications 2 à 15,
**caractérisé en ce que**
le radical alkyle comprend 6 à 50 atomes de carbone.

17. Procédé selon la revendication 16,
**caractérisé en ce que**
le radical alkyle comprend 8 à 20 atomes de carbone.

18. Procédé selon l'une des revendications 1 à 10, 11, 14 à 17,
**caractérisé en ce que**
l'additif est un éthoxylat d'alcool constitué d'un alcool monovalent comportant 8 à 20 atomes de C et 25 à 500 motifs d'oxyde d'éthylène.

19. Procédé selon les revendications 1 à 18,
**caractérisé en ce que**
le rapport des poids moléculaires de la partie hydrosoluble à la partie hydrophobe est de 10 à 50.

Fig.1

Fig.2

Fig.3

Fig.4

Fig.5

Fig.6

Fig.7

Fig.8

Fig.9

Fig.10

Fig.11

Fig.12

Fig.13

Fig.14

Fig.15

Fig.16

Fig.17

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19839054841
- WO 0012660 A2
- DE 102004058956
- DE 102005023762243
- DE 10323180 A1

**In der Beschreibung aufgeführte Nicht-Patentliteratur**

- Handbook of Industrial Surfactants. Gower Publishing